**Europäisches Patentamt**

**European Patent Office**

**Office européen des brevets**

(19)

(11) Veröffentlichungsnummer : **0 538 703 B1**

(12) **EUROPÄISCHE PATENTSCHRIFT**

(45) Veröffentlichungstag der Patentschrift :
28.06.95 Patentblatt 95/26

(21) Anmeldenummer : **92117392.8**

(22) Anmeldetag : **12.10.92**

(51) Int. Cl.⁶ : **G01N 33/569**, G01N 33/58, G01N 33/50

(30) Priorität : **25.10.91 CH 3128/91**

(43) Veröffentlichungstag der Anmeldung :
28.04.93 Patentblatt 93/17

(45) Bekanntmachung des Hinweises auf die
Patenterteilung :
28.06.95 Patentblatt 95/26

(84) Benannte Vertragsstaaten :
**BE CH DE DK ES FR GB GR IE IT LI LU NL PT**

(56) Entgegenhaltungen :
**JOURNAL OF IMMUNOLOGICAL METHODS
Bd. 44, Nr. 1, 1981, AMSTERDAM NL Seiten 73
- 78 P. GUOHUA ET AL 'Development of an
anticomplement immunoenzyme test for detection of EB virus nuclear antigen (EBNA)
and antibody to EBNA.'**

(54) Verfahren zum Nachweisen der An- oder Abwesenheit von Epstein-Barr-Virus Infektionen.

(56) Entgegenhaltungen :
**PROCEEDINGS OF THE NATIONAL ACADEMY
OF SCIENCE USA Bd. 75, Nr. 10, 1. Oktober
1978, WASHINGTON DC USA Seiten 5076 -
5080 K.S. ROSENTHAL ET AL. 'Translocation
of a hydrocarbon fluorescent probe between
Epstein-Barr virus and lymphoid cells: an assay for early events in viral infection.'**

(73) Patentinhaber : **INSTITUT VIRION AG
Weingartenstrasse 9
CH-8803 Rüschlikon (CH)**

(72) Erfinder : **Dobec, Marinko
21/V Bachtelstrasse
CH-8810 Horgen (CH)**

(74) Vertreter : **Troesch Scheidegger Werner AG
Patentanwälte,
Siewerdtstrasse 95,
Postfach
CH-8050 Zürich (CH)**

EP 0 538 703 B1

**Beschreibung**

Infektionen mit Epstein-Barr-Virus (EBV) sind gekennzeichnet durch das Aufscheinen verschiedener Antikörper, welche diagnostisch signifikant sind und mittels verschiedener EBV-Antigene und serologischer Tests nachgewiesen werden können.

In der EBV-Diagnostik ist es insbesondere erforderlich, zwischen akuten und vergangenen EBV-Infektionen differenzieren zu können.

EBV ist das ätiologische Agens von Mononukleosis infectiosa (MI) und ist weltweit verbreitet; 80 - 90% aller Erwachsenen wurden infiziert.

In Entwicklungsländern erfolgen die Primärinfektionen in den ersten 10 Lebensjahren. In den entwickelten Ländern erfolgen die Primärinfektionen üblicherweise bei jungen Erwachsenen.

Infektionen in der Kindheit sind meist asymptomatisch, doch selten durch die klassische MI begleitet. Im Gegensatz dazu zeigen 50 - 70% der jungen Erwachsenen bei primären EBV-Infektionen milde bis schwere Krankheit.

Von EBV wurde lange vermutet, dass es eine begleitende Rolle spielt in der Aetiologie von Burkitt's Lymphoma (vorwiegend ein Tumor von Kindern in Afrika und Neuginea) und Nasopharyngeal-Karzinom (undifferenziertes Squamos-Zell-Karzinom) des Nasopharynx, welches ein hohes Vorkommen unter südlichen Chinesen bei chronisch infizierten Individuen hat.

Für das Nachweisen von akuten und vergangenen EBV-Infektionen wurden bisher verschiedene serologische Methoden kombiniert, und es war häufig sogar noch notwendig zwischen Antikörpern der IgG- und IgM-Klasse zu unterscheiden. Aber auch die Verwendung verschiedener Tests führte nicht immer zu schlüssigen Resultaten. Zudem ist das gemeinsame Durchführen verschiedener serologischer Tests im Labor sehr aufwendig und kostenintensiv.

Im Rahmen der vorliegenden Erfindung wurde nun eine sehr einfache Methode gefunden, mit der man die An- oder Abwesenheit von akuten oder vergangenen EBV-Infektion nachweisen kann. Akute Infektionen sind gekennzeichnet durch die Anwesenheit von Antikörpern gegen das Hüllantigen von EBV, wogegen vergangene Infektionen durch die Anwesenheit von Antikörpern gegen das Kernantigen von EBV gekennzeichnet sind. Das Verfahren gemäss vorliegender Erfindung ermöglicht erstmals das Erkennen von Antikörpern gegen das Hüllantigen und gegen das Kernantigen von EBV in einem einzigen Testverfahren.

Die vorliegende Erfindung betrifft demnach ein Verfahren zum Erkennen der An- oder Abwesenheit von Antikörpern gegen das Hüllantigen und gegen das Kernantigen vom Epstein-Barr-Virus in einer biologischen Flüssigkeit mittels Fluoreszenz-Messung und fluoreszensmikroskopischer Auswertung, welches dadurch gekennzeichnet ist, dass man die Antikomplement-Immunfluoreszenstechnik sowie als Antigen P3HR-1-Zellen verwendet.

Die Antikomplement-Immunfluoreszenstechnik ist dem Fachmann geläufig und wird darüber hinaus auf Seite 33, dritter Absatz des Buches "Serologische Diagnose der Infektionskrankheiten" von Edwin H.-Lennette (Virion Edition, Cham, 1989) beschrieben. Die P3HR-1-Zellen sind bei der American Type Culture Collection (ATCC) unter der Nummer ATCC HTB 62 hinterlegt und dort jederzeit verfügbar.

Bei der fluoreszensmikroskopischen Auswertung verwendet man mit Vorteil ein Gegenfärbemittel insbesondere Evans-Blau.

Das Verfahren gemäss der vorliegenden Erfindung wird in einer biologischen Flüssigkeit, vorzugsweise in einer Serum- oder Plasmaprobe durchgeführt.

Das erfindungsgemässe Verfahren wird vorzugsweise so durchgeführt, dass man die biologische Flüssigkeit mit P3HR-1-Zellen, die auf einem Objektträger fixiert sind, inkubiert, ungebundene Antikörper durch Waschen entfernt und gebundene Antikörper durch Inkubieren des Antigen-Antikörper-Komplexes mit Komplement und anschliessendes Inkubieren mit einem Konjugat aus Antikomplement und einer fluoreszierenden Verbindung, Waschen von ungebundenem Konjugat und mittels fluoreszensmikroskopischer Auswertung nachweist.

Als Komplement kann jedes Säuger-Komplement verwendet werden, doch ist Meerschweinchenkomplement bevorzugt.

Das Antikomplement, welches mit einer fluoreszierenden Verbindung markiert ist, kann ebenfalls aus jeder Säugerart gewonnen werden, natürlich nicht aus derjenigen Säugerart, aus der man das Komplement gewonnen hat. Besonders geeignet sind hierbei Antikörper, insbesondere Ziegenantikörper, gegen Meerschweinchenkomplement. Besonders bevorzugt sind Ziegenantikörper gegen Meerschweinchen-C3-Komplement.

Als fluoreszierende Verbindung kommen diverseste Verbindungen in Betracht, doch ist Fluoreszeinisothiocyanat besonders bevorzugt.

Beispiel

a. benötigte Reagenzien

1) in vitro kultivierte P3HR-1-Zellen: vakuumverschlossene Glasröhrchen enthaltend 2 Objektträger, wobei jeder 6 Vertiefungen mit Aceton/Methanol-fixierten Zellen enthält.

2) Kontrollseren

(a) Hüll- und Kernantigen negativ;

(b) Hüllantigen positiv, Kernantigen negativ;

(c) Hüllantigen und Kernantigen positiv.

3) Komplement: lyophylisiertes Meerschweinchenserum

4) Konjugat: lyophylisierte mit Fluoreszeinisothiocyanat markierte Ziegenantikörper gegen Meerschweinchenkomplement C3.

5) Phosphat-gepuffte Kochsalzlösung von pH 7,3 ± 0,1

6) Evans-Blau: es wird eine Stock-Lösung 1:1000 (Gewicht/Volumen) verwendet.

b. Testdurchführung

1) Patientenserum oder Kontrollseren werden 1:10 in PBS verdünnt (0,01 + 0,09 ml), dicht verschlossen und bei 56° während 30 Minuten inaktiviert.

In die Vertiefungen der Objektträger werden 20 Mikroliter der verdünnten Seren pipetiert, wobei auch negative und positive Kontrollen mitgeführt werden.

2) Die Objektträger werden in eine gut geschlossenen feuchte Kammer gegeben und während 30 Minuten bei 37° inkubiert.

3) Die Serumverdünnungen werden von den Vertiefungen abgesaugt, worauf die Objektträger sorgfältig mit PBS gewaschen werden. Die Objektträger werden in eine Schale gegeben und zweimal während 5 Minuten mit PBS durchfeuchtet. Ueberschüssiger Puffer wird sorgfältig entfernt wobei ein vollständiges Austrocknen vermieden werden soll.

4) In alle Vertiefungen werden sofort 20 Mikroliter Meerschweinchenkomplement 1:10 in PBS verdünnt zugegeben (0,01 + 0,09, 0,02 + 0,18 usw.. entsprechend der Anzahl der Objektträger).

5) Es wird während 30 Minuten bei 37°C wie unter Punkt 2) inkubiert.

6) Während des Inkubierens stellt man die Menge an Konjugatlösung her, die man für die Anzahl von Objektträgern benötigt. Dies erfolgt anhand des folgenden Schemas:

| Anzahl Objektträger | PBS (ml) | Konjugat* (ml) | Evans-Blau Stocklösung (ml) | Schlussvolumen (ml) |
|---|---|---|---|---|
| 1 | 0.105 | 0.03 | 0.015 | 0.15 |
| 2 | 0.21 | 0.06 | 0.03 | 0.30 |
| 4 | 0.42 | 0.12 | 0.06 | 0.60 |
| 6 | 0.63 | 0.18 | 0.09 | 0.90 |

usw.

*Für den Fall, dass die Konjugatsarbeitslösung 1:5 ist.

7) Es wird wie unter Punkt 4) gewaschen.

8) Man gibt sofort 20 Mikroliter Konjugatverdünnung zu jeder Vertiefung.

9) Es wird während 30 Minuten bei 37° C wie unter Punkt 3) inkubiert.

10) Die Objektträger werden wie unter Punkt 4) behandelt.

11) Die Objektträger werden unter Zuhilfenahme eines Fluoreszensmikroskopes ausgewertet.

Als Fluoreszensmikroskop wird ein Standard-Fluoreszenz-Mikroskop mit einer 75W Xenonlampe, einem 490 nm Erreger- und 520 nm Sperr-Filter verwendet; es ist von Vorteil, ein Wasser- (oder Salz-)-immersionsobjektiv mit eingebautem Deckglas zu verwenden.

c. Die Interpretation der Resultate basiert auf dem Vorhandensein von (1) gefärbten Zytoplasma- und/oder Membranzellen des Hüllantigens (5-10% der Zellen) oder (2) gefärbten Kernantigenzellen (mehr als 90% der Zellen) und (3) der Abwesenheit jedwelcher Fluoreszenz im Falle eines negativen Resultates.

Positiver Test

(1) akute Infektion

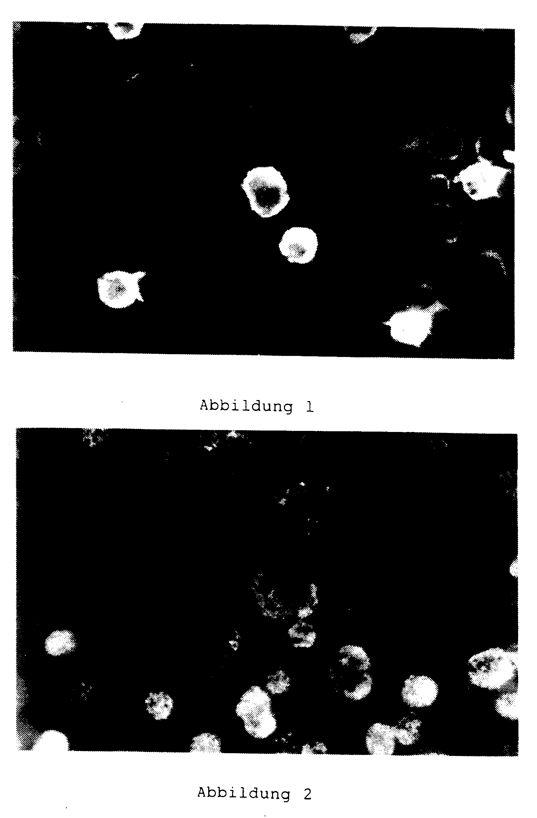

Antikörper gegen das Hüllantigen von EBV erzeugen ungefähr 5-10% fluoreszierende Zytoplasma- und/oder Membranzellen (vgl. Abbildung 1).

(2) Vergangene Infektion

Antikörper gegen das Kernantigen von EBV erzeugen ein Färben von mehr als 90% der Zellen (vgl. Abbildung 2).

Sowohl bei der akuten wie bei der vergangenen Infektion zeigt sich die Färbung als klar erkennbare brilliante Fluoreszenz.

(3) Negativer Test

Es kann keine Fluoreszenz beobachtet werden und alle Zellen erscheinen rot durch die Anwesenheit des Gegenfärbemittels Evans-Blau.

**Patentansprüche**

1.  Verfahren zum Erfassen von Epstein-Barr-Virus Infektionen in einer biologischen Flüssigkeit nach der Antikomplement-Immunfluoreszenztechnik mit anschliessender fluoreszenzmikroskopischer Auswertung, dadurch gekennzeichnet, dass man als einziges Antigen P3HR-1-Zellen verwendet, mit dem man Antikörper sowohl gegen das Hüllantigen wie gegen das Kernantigen von Epstein-Barr-Virus erkennen kann.

2.  Verfahren nach Anspruch 1, dadurch gekennzeichnet, dass man bei der fluoreszenzmikroskopischen Auswertung ein Gegenfärbemittel verwendet.

3.  Verfahren nach Anspruch 2, dadurch gekennzeichnet, dass man als Gegenfärbemittel Evans-Blau verwendet.

4.  Verfahren nach einem der Ansprüche 1-3, dadurch gekennzeichnet, dass man eine biologische Flüssigkeit mit P3HR-1-Zellen, die auf einem Objektträger fixiert sind, inkubiert, ungebundene Antikörper durch Waschen entfernt und gebundene Antikörper durch Inkubieren des Antigen-Antikörperkomplexes mit Komplement und anschliessendes Inkubieren mit einem Konjugat aus Antikomplement und einer fluoreszierenden Verbindung, Waschen von ungebundenem Konjugat und mittels fluoreszenzmikroskopischer Auswertung nachweist.

5.  Verfahren nach einem der Ansprüche 1-4, dadurch gekennzeichnet, dass man als biologische Flüssigkeit Serum oder Plasma verwendet.

6.  Verfahren nach Anspruch 3, dadurch gekennzeichnet, dass man als Komplement Meerschweinchenkomplement verwendet.

7.  Verfahren nach Anspruch 4, dadurch gekennzeichnet, dass man als Konjugat Antikörper, insbesondere Ziegenantikörper, gegen Meerschweinchenkomplement, die mit einer fluoreszierenden Verbindung markiert sind, verwendet.

8.  Verfahren nach Anspruch 7, dadurch gekennzeichnet, dass man als Antikörper gegen Meerschweinchen-C3-Komplement Ziegenantikörper verwendet.

9.  Verfahren nach Anspruch 8, dadurch gekennzeichnet, dass man als fluoreszierende Verbindung Fluoreszeinisothiocyanat verwendet.

**Claims**

1.  Method for the detection of Epstein-Barr virus infections in a biological liquid by the anticomplement immunofluorescence technique with subsequent fluorescence microscopic evaluation, characterised in that P3HR-1 cells with which antibodies both against the coat antigen and against the core antigen of Epstein-Barr virus can be identified are used as sole antigen.

2. Method according to Claim 1, characterised in that a counterstain is used in the fluorescence microscopic evaluation.

3. Method according to Claim 2, characterised in that Evans blue is used as counterstain.

4. Method according to any of Claims 1-3, characterised in that a biological liquid is incubated with P3HR-1 cells which are immobilised on a slide, unbound antibodies are removed by washing, and bound antibodies are detected by incubation of the antigen-antibody complex with complement and subsequent incubation with a conjugate composed of anticomplement and of a fluorescent compound, washing of unbound conjugate and evaluation by means of fluorescence microscopy.

5. Method according to any of Claims 1-4, characterised in that serum or plasma is used as biological liquid.

6. Method according to Claim 3, characterised in that guinea pig complement is used as complement.

7. Method according to Claim 4, characterised in that antibodies, in particular goat antibodies, against guinea pig complement which are labelled with a fluorescent compound are used as conjugate.

8. Method according to Claim 7, characterised in that goat antibodies are used as antibodies against guinea pig C3 complement.

9. Method according to Claim 8, characterised in that fluorescein isothiocyanate is used as fluorescent compound.

## Revendications

1. Procédé pour la détection d'infections dues au virus de Epstein-Barr dans un fluide biologique, selon la technique d'immunofluorescence d'anticomplément avec évaluation subséquente au microscope à fluorescence, caractérisé en ce qu'on utilise, comme seul antigène, les cellules P3HR-1 permettant de reconnaître des anticorps aussi bien contre l'antigène de l'enveloppe que contre l'antigène du noyau du virus de Epstein-Barr.

2. Procédé selon la revendication 1, caractérisé en ce que l'on utilise, dans le cas de l'évaluation au microscope à fluorescence, un agent colorant de contraste.

3. Procédé selon la revendication 2, caractérisé en ce qu'on utilise, en tant qu'agent colorant de contraste, le bleu de Evans.

4. Procédé selon l'une des revendications 1-3, caractérisé en ce que l'on soumet à incubation un fluide biologique avec des cellules P3HR-1, qui sont fixées sur une lamelle, on élimine les anticorps non liés, par lavage, et on décèle les anticorps liés par incubation du complexe antigène-anticorps avec un complément et, subséquemment, incubation avec un conjugué formé de l'anticomplément et d'un composé fluorescent, lavage du conjugué non lié et évaluation au microscope à fluorescence.

5. Procédé selon l'une quelconque des revendications 1-4, caractérisé en ce qu'on utilise, en tant que fluide biologique, du sérum ou du plasma.

6. Procédé selon la revendication 3, caractérisé en ce qu'on utilise, en tant que complément, un complément de cochon d'Inde.

7. Procédé selon la revendication 4, caractérisé en ce qu'on utilise, en tant que conjugué, des anticorps, en particulier des anticorps de chèvre contre le complément de cochon d'Inde, qui sont marqués au moyen d'un composé fluorescent.

8. Procédé selon la revendication 7, caractérisé en ce qu'on utilise, en tant qu'anticorps contre le complément C3 du cobaye, un anticorps de chèvre.

9. Procédé selon la revendication 8, caractérisé en ce qu'on utilise, en tant que composé fluorescent, de l'isothiocyante de fluorescéine.

Abbildung 1

Abbildung 2